# EUROPEAN PATENT SPECIFICATION

(11) **EP 1 626 082 B1**
(45) Date of publication and mention of the grant of the patent: **16.01.2008**
(21) Application number: 04254885.9
(22) Date of filing: 13.08.2004
(51) Int. Cl.: C12M 1/00, C12M 1/10, G01N 35/00, B01F 11/00

(54) **Shaking system for cell culture incubator**
Rüttler für Zellkulturklimaschrank
Systeme de vibration pour incubateur de culture cellulaire

(43) Date of publication of application: 15.02.2006
(73) Proprietor: The Automation Partnership (Cambridge) Limited, Royston, Hertfordshire SG8 5WY (GB)
(72) Inventor: Malinge, David S., Royston Hertfordshire SG8 9DE (GB)
(74) Representative: Brunner, Michael John

(56) References cited:
- EP-A1- 1 445 307
- EP-A2- 0 569 214
- US-A- 5 735 587

## Description

The invention relates to a system for stirring or shaking vessels which, for example, contain cells which are processed in an incubator of a cell culture system.

Many different types of shaking systems exist. For example, figure-of-eight shakers move in a figure-of-eight pattern and may switch smoothly between clockwise and counterclockwise motion. Hand-motion shakers duplicate the up and down action associated with a handshake. In another type, the entire platform of an orbital shaker moves in a circular orbit. These shakers have many applications in molecular biology including aeration and prevention of "skin" formation. Platforms on reciprocating shakers make back-and-forth "sloshing" motions required for some test procedures. It is known to provide shakers with both an orbital (eccentric) and a reciprocating motion for extra mixing versatility. Platform shakers may provide either a rocking "see-saw" motion or a consistent, three-dimensional rocking motion. Rotating shakers spin samples in test tubes, flasks, or bottles. Wrist-motion shakers mimic the side-to-side action of hand mixing.

EP-A-0569214 describes a prior art shaker wherein samples can be shaken and, whilst being shaken are simultaneously automatically rotated (precessed) slowly around an upright axis.

For use in incubators, platform shakers are often used, providing one or more trays or shelves on which cell culture vessels can be placed within the incubator, the trays or shelves being capable of being independently shaken. However, such incubators, while they are capable of handling plural cell culture vessels, require manual loading or unloading of the vessels and there is a need for an incubator shaking system which can be loaded robotically.

According to a first aspect of the present invention there is provided an incubator for example for a cell culture system, the incubator being arranged to handle a plurality of vessels, the incubator including a rotatable support defining a plurality of support locations for vessels at predefined positions around an upright axis and being rotatable around the upright axis to move the vessels between a number of positions about the axis; a drive transmission system for connecting the support to a drive motor, whereby the support can be rotated to a desired position for loading or unloading of a vessel to or from a respective support location; and a drive mechanism operable to move each support location eccentrically about an axis independently of the rotation of the support about the upright axis, whereby vessels disposed on the support can be shaken.

The vessels may be cell culture vessels in a cell culture system.

The support locations are independently eccentrically movable, for example, about individual axes parallel to the upright axis, or alternatively the support is eccentrically movable about the upright axis of the rotatable support whereby the support locations are simultaneously eccentrically movable.

A plurality of independently rotatable supports may be provided, each connectable to the or a respective drive motor. The or each support may be configured to receive vessels in two rings substantially coaxial with the upright axis, one within the other. The rings may be independently rotatable relative to one another.

A plurality of supports can be disposed one above another.

A robotic handling device is preferably arranged to move vessels on to or remove vessels from the or each support as required.

Such an incubator is capable of carrying out the required shaking of multiple culture flasks or vessels as desired and an individual flask may even be omitted from the shaking process by using a robotic handling device to lift the particular flask from its support location during the shaking process.

An example of an incubator according to the present invention will now be described with reference to the accompanying drawings in which:-

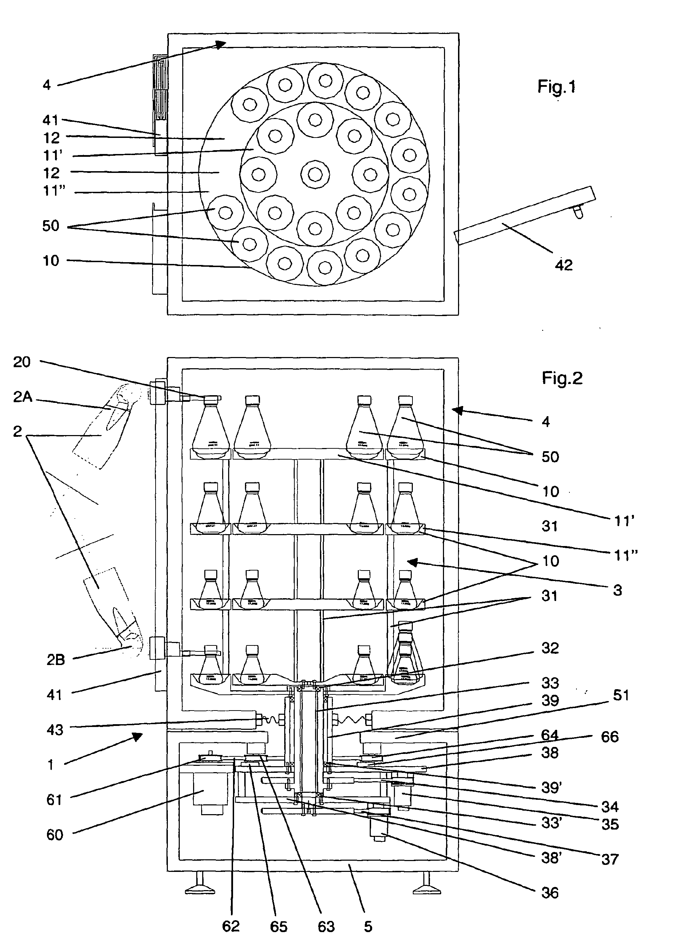

Figures 1 & 2 illustrate an incubator for a cell culture system in plan view and side elevation respectively, both in part section.

The incubator 1 shown in the drawing includes a housing 4 in order to enable a controlled environment to be maintained and adjusted. The incubator housing 4 includes a slidable access panel or door 41 through which a robotic arm 2 with a gripper unit 20 may be extended to load/unload culture vessels (Erlenmayer flasks) 50. On the opposite side, the housing has an operator access door 42. The robotic arm 2 is shown in both a top 2A and a bottom 2B position to show the range of vertical movement possible. For simplicity further details of the housing of the incubator are not shown in the drawings.

The incubator 1 includes a plurality of rotatable supports 10 in the form of pairs of rotatable rings 11', 11" defining, around their common axis, a series of support locations 12 for the culture vessels or flasks 50 in what is, effectively, a carousel 3. The vessels or flasks 50 may be of different sizes as shown on the different supports 10.

The plural inner rings 11' are fixed for movement together as are the plural outer rings 11 ". Upright frame elements 31 extend between adjacent rings 11', 11" to support one above another for movement together. The lowest support 10 includes a bearing 32 which allows the inner ring 11' to rotate within the outer ring 11 ", carrying with it the rings disposed above it. A first drive shaft 33 is driven through a transmission 34 by a motor 35 to rotate the inner set of rings 11' of the carousel 3 for loading/unloading of culture vessels or flasks 50. A second, tubular, drive shaft 39 surrounds the first drive shaft 33 and is driven through a transmission 37 by a motor 36 to rotate the outer set of rings 11" of the carousel 3 for loading/unloading of culture vessels or flasks 50. The drive shafts 39 and 33 are supported on respective bearings 39', 33' on upper and lower supporting bracket members 38, 38' respectively.

A bellows type flexible seal 43 seals the bottom of the housing 4 allowing the drive mechanisms and motors to be disposed in a separate, lower housing 5 disposed below and supporting the incubator 4. This separation simplifies the task of maintaining the required conditions within the incubator 1.

To shake or vibrate culture vessels or flasks 50 disposed on the supports 10 in the carousel 3, a drive motor 60 is connected via a pulley 61 to a drive belt 62 which is also passed around eccentrically mounted pulleys 63, 64 fixed on the underside of the top member 51 of the lower housing 5 and having respective eccentrics 65,66 attached to the upper support bracket member 38 to support the flask supports 10 for eccentric movement relative to the incubator housing 4 under the action of the motor 60.

To improve access to the inner rings 11', the outer rings 11" are preferably loaded with flasks 50 in such a manner as to leave at least one support position 12 unoccupied (as best seen in Figure 1A).

If the amplitude of shaking is significant in terms of positioning of the support locations 12 and a robotic arm (as per the example) is utilised, then it may be necessary or desirable to determine or fix the position of the support locations when the supports are stationary, before a vessel can be loaded into a support location or removed therefrom by the gripper of the robotic arm. Determining the position may be achieved by means of a suitable sensor, eg. an inductive sensor or a shaft encoder connected to the motor 60 and the robotic arm may be supplied with data indicative of the position of the support location from the sensor. Alternatively, for example, the supports may be arranged to be brought automatically to a predetermined stop position using feedback from a sensor to a control system operating the motor 60. A further alternative is to provide a biassing system to move the supports to the predetermined position from whatever position they have stopped at.

## Claims

1. An incubator (1) arranged to handle a plurality of vessels (50), the incubator including:
a rotatable support (10) defining a plurality of support locations (12) for vessels at predefined positions around an upright axis and being rotatable around the upright axis to move the vessels between a number of positions about the axis;
a drive transmission system (34) for connecting the support to a drive motor (35), whereby the support can be rotated to a desired position for loading or unloading of a vessel to or from a respective support location; and
a drive mechanism (60-66) operable to move each support location (12) eccentrically about an axis independently of the rotation of the support (10) about the upright axis, whereby vessels disposed on the support can be shaken.

2. An incubator according to claim 1, wherein the support locations (12) are independently eccentrically movable.

3. An incubator according to claim 2, wherein the support locations (12) are independently eccentrically movable about individual axes parallel to the upright axis.

4. An incubator according to claim 1, wherein the support (10) is eccentrically movable about the upright axis of the rotatable support whereby the support locations (12) are simultaneously eccentrically movable.

5. An incubator according to any of claims 1 to 4, including a plurality of independently rotatable supports (10,11,11') each connectable to the or a respective drive motor (35,36).

6. An incubator according to any of claims 1 to 5, wherein the or each support (10) is configured to receive vessels in two rings (11,11') substantially coaxial with the upright axis, one within the other.

7. An incubator according to claim 6, wherein the rings (11,11') are independently rotatable relative to one another.

8. An incubator according to any of claims 1 to 7, comprising a plurality of supports (10) disposed one above another.

9. An incubator according to any of claims 1 to 8, further including a robotic handling device (2,20) arranged to move vessels (50) on to or remove vessels from the or each support (10) as required.

10. An incubator according to any of claims 1 to 9, further including a sensor for sensing the position of a rotatable support when it is stationary.

11. An incubator according to any of claims 1 to 9, further including means for causing a rotatable support (10) to adopt a predetermined position when the shaking drive mechanism is stopped in use.

12. A cell culture system including an incubator (1) according to any of claims 1 to 11.

13. A cell culture system according to claim 12, including a plurality of cell culture vessels (50).

## Patentansprüche

1. Inkubator (1), angeordnet, um eine Vielzahl von Gefässen (50) zu behandeln, wobei der Inkubator umfasst:
- einen drehbaren Träger (10), der eine Vielzahl von Trageorten (12) für Gefässe an vordefinierten Positionen um eine aufrechte Achse definiert und welcher um die aufrechte Achse drehbar ist, um die Gefässe zwischen einer Anzahl von Positionen um diese Achse zu bewegen;
- ein Antriebs-Übertragungs-System (34), zum Verbinden des Trägers mit einem Antriebs-Motor (35), wobei der Träger in eine gewünschte Position zum Beladen oder Entladen eines Gefässes zu oder von einem jeweiligen Trageort gedreht werden kann; und
- einen Antriebs-Mechanismus (60-66), der betätigbar ist, um jeden Trageort (12) exzentrisch um eine Achse unabhängig von der Drehung des Trägers (10) um die aufrechte Achse zu bewegen, wobei die auf dem Träger angeordneten Gefässe gerüttelt werden können.

2. Inkubator gemäss Anspruch 1, bei dem die Trageorte (12) unabhängig voneinander exzentrisch bewegbar sind.

3. Inkubator nach Anspruch 2, bei dem die Trageorte (12) unabhängig voneinander exzentrisch um individuelle Achsen, parallel zur aufrechten Achse, bewegbar sind.

4. Inkubator gemäss Anspruch 1, bei dem der Träger (10) in exzentrischer Weise um die aufrechte Achse des drehbaren Trägers bewegbar ist, wobei die Trageorte (12) simultan exzentrisch bewegbar sind.

5. Inkubator gemäss einem der Ansprüche 1 bis 4, umfassend eine Vielzahl von unabhängig drehbaren Trägern (10, 11, 11'), wobei jeder mit dem oder einem jeweiligen Antriebs-Motor (35, 36) verbindbar ist.

6. Inkubator gemäss einem der Ansprüche 1 bis 5, bei dem der oder jeder Träger (10) ausgestaltet ist, um Gefässe in zwei Ringen (11, 11') substantiell koaxial mit der aufrechten Achse, einer innerhalb des anderen, aufzunehmen.

7. Inkubator gemäss Anspruch 6, bei dem die Ringe (11, 11') unabhängig voneinander drehbar sind.

8. Inkubator gemäss einem der Ansprüche 1 bis 7, umfassend eine Vielzahl von Trägern (10), die übereinander angeordnet sind.

9. Inkubator gemäss einem der Ansprüche 1 bis 8, weiterhin umfassend eine robotische Betätigungs-Einrichtung (2, 20), die angeordnet ist, um Gefässe (50) auf oder Gefässe von dem oder jedem Träger (10) hin zu bewegen, beziehungsweise weg zu bewegen, wie erforderlich.

10. Inkubator gemäss einem der Ansprüche 1 bis 9, weiterhin umfassend einen Sensor zum Feststellen der Position eines drehbaren Trägers, wenn dieser stationär ist.

11. Inkubator gemäss einem der Ansprüche 1 bis 9, weiterhin umfassend Mittel zum Bewirken, das eine drehbare Stütze eine vorbestimmte Position einnimmt, wenn der Rüttel-Antriebs-Mechanismus beim Einsatz gestoppt ist.

12. Zellkultur-System, umfassend einen Inkubator (1) gemäss einem der Ansprüche 1 bis 11.

13. Zellkultur-System gemäss Anspruch 12, umfassend eine Vielzahl von Zellkultur-Gefässen (50).

## Revendications

1. Incubateur (1), arrangé pour actionner une pluralité de récipients (50), où l'incubateur comprend:
- un support (10) rotatif, définissant une pluralité d'emplacements de support (12) pour des récipients à des positions prédéfinies autour d'un axe vertical et pouvant être tourné autour de l'axe vertical pour déplacer les récipients entre un nombre de positions autour de cet axe;
- un système d'entrainement de transmission (34) pour connecter le support à un moteur d'entrainement (35), où le support peut être tourné dans une position désirée pour charger ou décharger un récipient envers ou d'un endroit de support respectif; et
- un mécanisme d'entrainement (60, 66), opératif pour déplacer chaque emplacement de support (12) d'une manière excentrique autour d'un axe indépendamment de la rotation du support (10) autour de l'axe vertical, où les récipients disposés sur le support peuvent être vibrés.

2. Incubateur selon la revendication 1, où les emplacements de support (12) peuvent être déplacés indépendamment d'une manière excentrique.

3. Incubateur selon la revendication 2, où les emplacements de support (12) peuvent être déplacés indépendamment d'une manière excentrique autour de l'axe individuel, parallèle à l'axe vertical.

4. Incubateur selon la revendication 1, où le support (10) peut être déplacé d'une manière excentrique autour de l'axe vertical du support rotatif, où les emplacements de support (12) peuvent être déplacés d'une manière excentrique simultanément.

5. Incubateur selon l'une des revendications 1 à 4, comprenant une pluralité de supports rotatifs (10, 11, 11') indépendants, qui sont chacun à connecter au moteur d'entrainement (35, 36) respectif.

6. Incubateur selon l'une des revendications 1 à 5, où le ou chaque support (10) est configuré pour recevoir des récipients dans deux anneaux (11, 11') qui sont essentiellement coaxiaux avec l'axe vertical, l'un à l'intérieur de l'autre.

7. Incubateur selon la revendication 6, où les anneaux (11, 11') peuvent être tourné indépendamment, relatif l'un par rapport à l'autre.

8. Incubateur selon l'une des revendications 1 à 7, comprenant une pluralité de supports (10), disposés l'un au dessus de l'autre.

9. Incubateur selon l'une des revendications 1 à 8, comprenant aussi un système de traitement robotique (2, 20), arrangé pour déplacer les récipients (50) sur le ou chaque support, ou les enlever du ou de chaque support (10) au besoin.

10. Incubateur selon l'une des revendications 1 à 9, comprenant aussi un détecteur pour détecter la position d'un support rotatif, quand il est stationnaire.

11. Incubateur selon l'une des revendications 1 à 9, comprenant aussi des moyens pour emmener le support rotatif (10) pour adapter une position prédéterminée, quand le mécanisme d'entrainement de vibration est stoppé lors de l'utilisation.

12. Un système de culture cellulaire, comprenant un incubateur (1) selon l'une des revendications 1 à 11.

13. Un système de culture cellulaire selon la revendication 12, comprenant une pluralité de récipients (50) de culture cellulaire.
